# EUROPEAN PATENT APPLICATION

(11) **EP 1 428 543 A1**
(43) Date of publication of application: **16.06.2004**
(21) Application number: 02724745.1
(22) Date of filing: 09.05.2002
(51) Int. Cl.: A61M 5/315

(54) **INJECTOR**

(30) Priority: 06.06.2001 JP 2001170756; 05.04.2002 JP 2002104334
(71) Applicant: KABUSHIKI KAISHA TOP, Tokyo 120-0035 (JP)
(72) Inventor: CHIBA, Atsushi, c/o KABUSHIKI KAISHA TOP, Tokyo 120-0035 (JP); ICHIKAWA, Kazuhiro, c/o KABUSHIKI KAISHA TOP, Tokyo 120-0035 (JP)
(74) Representative: Fenlon, Christine Lesley
(86) International application number: PCT/JP2002/004519
(87) International publication number: WO 2002/100464

(57) **Abstract**

A injector provided with a gasket having an excellent adhesion to an inner wall surface of a cylindrical barrel, the injector having a reduced overall length as well as a good manufacturability. The injector is provided with a gasket 6 which slides within the cylindrical barrel 4 while tightly contacting the inner wall surface 4a of the cylindrical barrel 4. The gasket 6 is an a substantial discoid of which the whole side face is a curved surface 11 having a convex arc towards the inner wall surface 4a along the plunger 5, and tightly contacts the inner wall surface 4a of the cylindrical barrel 4 at a portion of the curved surface 11. The gasket 6 is joined to a base 10 provided on the tip of a plunger 5 via a hole 12 while leaving a flexible space on the side of the tip of the plunger 5. An annular groove portion 13 having a larger diameter than the base 10 is provided on the surface of the plunger 5 side, and a bending portion 14 on the outer peripheral side of the annular groove portion. When the gasket 6 slides along the axial of the cylindrical barrel 4, the bottom 13a of the annular groove portion 13 becomes a supporting point and the bending portion 14 bends towards the axial of the plunger 5. The bend portion 14 is formed with smaller distance between the bottom 13a of the annular groove portion 13 and the peak P of the curved surface 11 than the curvature radius of the curved surface 11. The gasket 6 is made of synthetic resin having the Shore (A) hardness from 20 to 70.

## Description

### FIELD OF THE INVENTION:

The present invention relates to a injector made of synthetic resin.

### BACKGROUND ART

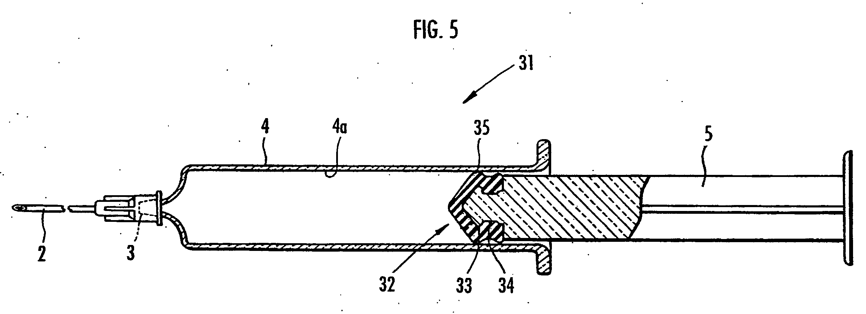

Conventionally, for example, a synthetic resin injector 31 as shown in FIG. 5 is generally known as single-use (disposable) injectors. The injector 31 consists of a cylindrical barrel 4 made of synthetic resin having a luer taper portion 3 to which a needle 2 is to be attached and a plunger 5 made of synthetic resin having a gasket 32 on its tip which slides while tightly contacting the inner wall surface 4a of the cylindrical barrel 4. The plunger 5 is made of polypropylene, polystyrene, polycarbonate or the like, and a crown-like projecting portion 34 having a bulge portion 33 which bulges to the outer peripheral side is formed on the tip of the plunger 5.

The gasket 32 is formed from elastic resin in a shape similar to the projecting portion 34. The gasket 32 fits into the projecting portion 34 to form a sliding portion 35 which corresponds to the bulge portion 33 and tightly contacts the inner wall surface 4a of the cylindrical barrel 4. In the injector, the gasket 32 has a risk of dropping off from the plunger 5 since the pressure inside the injector becomes negative when a medical fluid is taken into the injector. However, the sliding portion 35 of the gasket 32 is engaged with the bulge portion 33, and thus, the gasket 32 may be prevented from dropping off against the negative pressure.

In the meantime, the overall length of the conventional injector 31 shown in FIG. 5 is disadvantageously increased as the projecting portion 34 is formed in a crown-like shape in order to provide a configuration for preventing the gasket 32 from dropping off. In addition, since the conventional injector 31 has such a configuration that the gasket 32 fits into the projecting portion 34 of the plunger 5, both of which are made of different materials as mentioned above, there has been a problem that the gasket 32 and the plunger 5 should be formed separately and assembled together in a post-process.

### DISCLOSURE OF THE INVENTION

It is an object of the present invention to solve such problems and to provide a injector having a gasket with excellent adhesion to the inner wall surface of the cylindrical barrel, the injector having a reduced overall length as well as a good manufacturability.

To achieve aforementioned object, the injector to be used in the present invention is a injector consisting of a cylindrical barrel to which a needle is to be attached, and a plunger having a gasket on the tip, which slides while tightly contacting the inner wall surface of the injector, wherein the gasket is a substantial discoid form of which the whole side face is a curved surface having a convex arc towards the inner wall surface along the axial direction of the plunger, and tightly contact the inner wall surface of the cylindrical barrel at a portion of the curved surface, the gasket is joined to a base provided on the tip of the plunger via a hole provided on an end face of the gasket facing the plunger while leaving a flexible space on the plunger side, the base having a smaller diameter than the end face of the plunger, an annular groove portion having a larger diameter than the base is provided on the surface of the plunger side, and a bending portion is provided on the outer peripheral side of the annular groove portion in which the bottom of the annular groove portion becomes a supporting point when the gasket slides along the axial direction of the cylindrical barrel while tightly contacting the inner wall surface of the cylindrical barrel, and the bending portion bends towards the axial of the plunger, and the bend portion is formed with smaller distance between the bottom of the annular groove portion and the peak of the curved side than the curvature radius of the curved side.

According to the injector of the present invention, the gasket being a substantial discoid fits into the base provided on the tip of the plunger via the hole. Therefore, it is not necessary to form the tip of the plunger in crown-like shape to engage with the crown-like portion, so that the overall length can be decreased.

According to the injector of the present invention, the gasket has a curved side face generally along the axial direction of the plunger comprising a convex arc against the inner wall surface of the cylindrical barrel, a portion of the curved surface tightly contacting the inner wall surface of the cylindrical barrel. Furthermore, the gasket has an annular groove portion having a larger diameter than the base on the surface of the plunger side, and a bending portion on the outer peripheral side of the annular groove portion. When the gasket slides along the axial of the cylindrical barrel while tightly contacting the inner wall surface of the cylindrical barrel, the bottom of the annular groove portion becomes a supporting point and the bending portion bends towards the axial of the plunger.

Consequently, the bending portion bends towards the side opposed to the plunger due to the negative pressure inside the injector when a medical fluid is taken into the injector. The bending portion bends towards the plunger side due to the positive pressure which occurs when a medical fluid is discharged.

At this point, the gasket is joined to the base while leaving a flexible space on the plunger side between the gasket and the plunger. Therefore, when the gasket slides towards the needle to discharge a medical fluid and the bending portion bends towards the plunger side, the bending portion can smoothly bend towards the plunger side on the outer peripheral side of the base.

As the result, when the plunger slides along the inner wall surface of the cylindrical barrel, the gasket is not affected by a creep deformation since the gasket bends at a portion which differs from the normal bending portion and tightly contacts the inner wall surface of the cylindrical barrel. Moreover, the gasket obtains an excellent adhesion to the inner wall surface of the cylindrical barrel since the strength of adhesion between the gasket and the inner wall surface of the cylindrical barrel is increased by bending.

Although the annular groove portion may be formed on the end face of the gasket facing the needle, such a configuration may have a risk that the chemical remains in the annular groove portion when it is discharged. However, by forming the annular groove portion on the side of the gasket facing the plunger according to the present invention, the annular groove portion does not contact the chemical so that residue of the chemical in the annular groove portion can be avoided.

The gasket is preferably made of synthetic resin having Shore (A) hardness from 20 to 70 so as to be able to ensure the adhesion of the bending portion to the inner wall surface of the cylindrical barrel and to sensitively respond to the negative pressure caused by sucking or the positive pressure caused by discharging. When the Shore (A) hardness is less than 20, the gasket is likely to cause a creep deformation and there are some cases that enough adhesion to the inner wall surface of the cylindrical barrel cannot be obtained. On the contrary, when the Shore (A) hardness is more than 70, the flexibility of the bending portion is decreased and it can be difficult to sensitively respond to the negative pressure caused by sucking or the positive pressure caused by discharging.

### BRIEF DESCRIPTION OF THE DRAWINGS

FIG. 1 is an explanatory sectional view showing a constitutional example of the injector according to the present invention;
FIG. 2 is an enlarged sectional view showing the gasket shown in FIG. 1;
FIG. 3 is an operational schematic explanation of the injector shown in FIG. 1; and
FIG. 4 is an explanatory sectional view showing an example of a manufacturing method of the injector shown in FIG. 1.
FIG.5 is an explanatory sectional views showing a constitutional example of a conventional injector.

### BEST MODE FOR CARRYING OUT THE INVENTION

Referring now to the attached figures, the preferred embodiment of the present invention will be further described below.

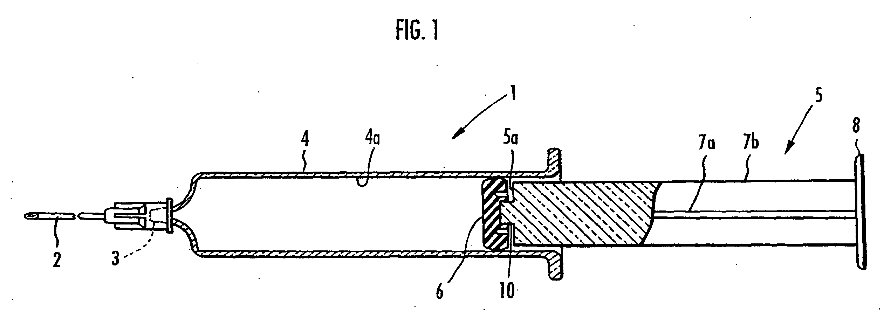

As shown in FIG. 1, a injector 1 of the present embodiment consists of a cylindrical barrel 4 made of a synthetic resin having a luer taper portion 3 to which a needle 2 is attached, and a plunger 5 made of a synthetic resin. A gasket 6 which slides within the cylindrical barrel while tightly contacting an inner wall surface 4a of the cylindrical barrel 4 is provided on the tip 5a of the plunger 5.

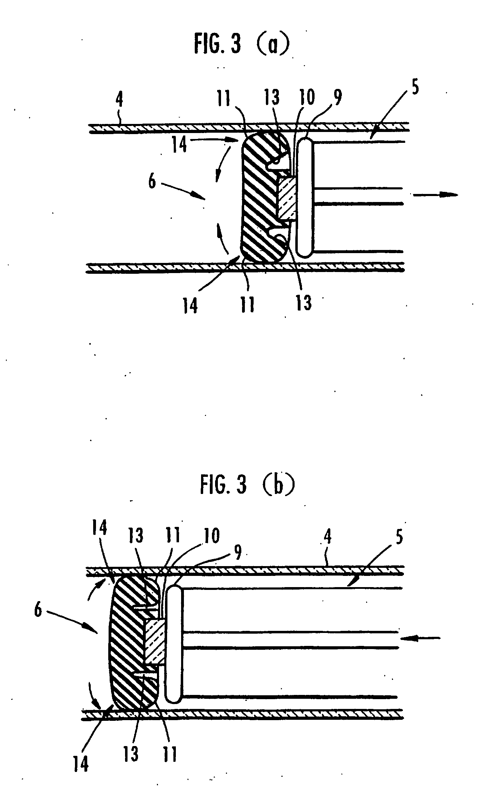

The plunger 5 is made of, for example, polypropylene synthetic resin, and is provided with a circular plate 8 as a fingerhold on the back end of plates 7a and 7b which were assembled in a crossed shape, and a discoid flange 9 on the tip of the plunger 5 (see FIG. 3). On the flange 9, a base 10 having a smaller diameter than that of the flange 9 is provided. The gasket 6 is formed to be joined to the base 10.

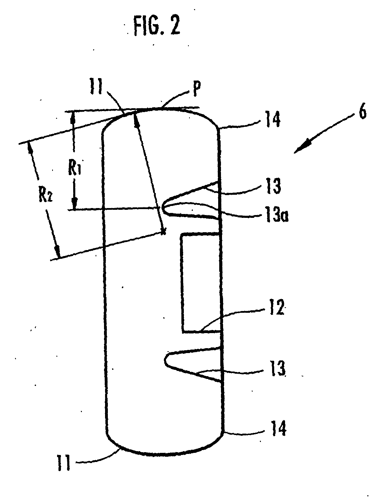

The gasket 6 is in a substantial discoid form, and as it is enlarged in FIG. 2, the whole side face is a curved surface 11 having a convex arc towards the inner wall surface 4a of the cylindrical barrel 4 along the axial of the plunger 5. The gasket 6 fits into the base 10 of the plunger 5 via a hole 12 provided on an end face of the gasket 6 facing the plunger 5, and the gasket 6 is joined to the base 10 under the circumstances. Here, a flexible space is provided between the gasket 6 and the flange 9 of the plunger 5 so that the gasket 6 can bend towards the tip of the plunger 5.

Moreover, on the end face of the gasket 6 which is facing the plunger 5, an annular groove portion 13 is provided on the outer peripheral side of the hole 12 which is to fit into the base 10. Further peripheral side of the annular groove portion 13 is the bending portion 14.

When the gasket 6 slides along the inner wall surface 4a of the cylindrical barrel 4, the bottom of the annular groove portion becomes a supporting point and the bending portion 14 freely bends towards the axial of the plunger 5. The gasket 6 is formed so that the distance R₁ between the annular groove portion 13 and a peak P of the curved surface 11 is smaller than the curvature radius R₂ of the curved surface 11.

Preferably the gasket 6 has the Shore (A) hardness ranging from 20 to 70 measured by spring type hardness tester (type A) standardized by JIS (Japanese Industrial Standards) K6301. Since the gasket 6 has hardness within the range, an adhesion of the bending portion 14 to the inner wall surface 4a is ensured. Moreover, the bending portion 14 can sensitively respond to the negative pressure caused by sucking or the positive pressure caused by discharging.

The Shore (A) hardness may be suitably selected within the range based on the volume of the injector 1. For the injector 1 having a small diameter with nominal size from 1 to 3 ml, a soft gasket is preferred. On the contrary, for the injector 1 having a large diameter with nominal size from 30 to 100 ml, a hard gasket is preferred.

As the resin having the Shore (A) hardness within the range, for example, thermoplastic elastomer such as polystyrene-based thermoplastic elastomer, polyolefin-based thermoplastic elastomer, polyester-based thermoplastic elastomer, polyamide-based thermoplastic elastomer and polyvinylchloride-based thermoplastic elastomer, and polystyrene-added styrene-butadiene rubber (SBR) may be used.

As the polystyrene-based thermoplastic elastomer, for example, styrene-ethylene/propylene-styrene block copolymer (S-EP-S), styrene-ethylene/butylene-styrene block copolymer (S-EB-S), styrene-butadiene-styrene block copolymer, and styrene-isoprene-styrene block copolymer may be recited.

Referring now to FIG. 3, the operation of the gasket 6 will be described.

First, as shown in FIG. 3(a), a medical fluid is taken into the cylindrical barrel 4 of the injector 1 by pulling the plunger 5 in the arrow direction. At this point, since the pressure inside the cylindrical barrel 4 becomes negative, the bending portion 14 of the gasket 6 bends towards the needle 2 side. As the result, the gasket 6 tightly contacts the inner wall surface 4a of the cylindrical barrel 4 at a portion which is closer to the plunger 5 than the peak P of the curved surface 11.

Then, as shown in FIG. 3(b), the chemical inside the cylindrical barrel 4 of the injector 1 is discharged by pushing the plunger 5 in the arrow direction. At this point, since the gasket 6 receives positive pressure, the bending portion 14 bends towards the plunger 5 side.

The base 10 has a smaller diameter than that of the flange 9, and the gasket 6 is joined to the base 10 while leaving a space between the flange 9 and the gasket. Therefore, when the chemical is discharged, the bending portion 14 can bend towards the base 10 as if it slips into the outer peripheral side of the base 10. As the result, the gasket 6 tightly contacts the inner wall surface 4a at a portion which is farther from the plunger 5 than the peak P of the curved surface 11.

Under normal conditions, the gasket 6 adheres to the inner wall surface 4a of the cylindrical barrel 4 at the peak P of the curved surface 11. However, when the gasket 6 slides along the inner wall surface 4a of the cylindrical barrel 4, the gasket 6 tightly contacts the inner wall surface 4a of the cylindrical barrel 4 at a portion which is closer to or farther from the plunger 5 than the peak P as mentioned above. That is, when the gasket 6 is sliding, it tightly contacts the inner wall surface 4a at a portion different from the portion contacting the inner wall surface 4a under normal conditions.

Therefore, even if a creep deformation occurs during storage, the gasket 6 can obtain an excellent adhesion to the inner wall surface 4a of the cylindrical barrel 4 regardless of the creep deformation.

In addition, the gasket 6 increases the adhesion strength by tightly contacting the inner wall surface 4a of the cylindrical barrel 4 at a portion different from the peak P. Therefore, the gasket 6 can adhere to the inner wall surface 4a of the cylindrical barrel 4 more tightly so as to be able to achieve an excellent adhesion.

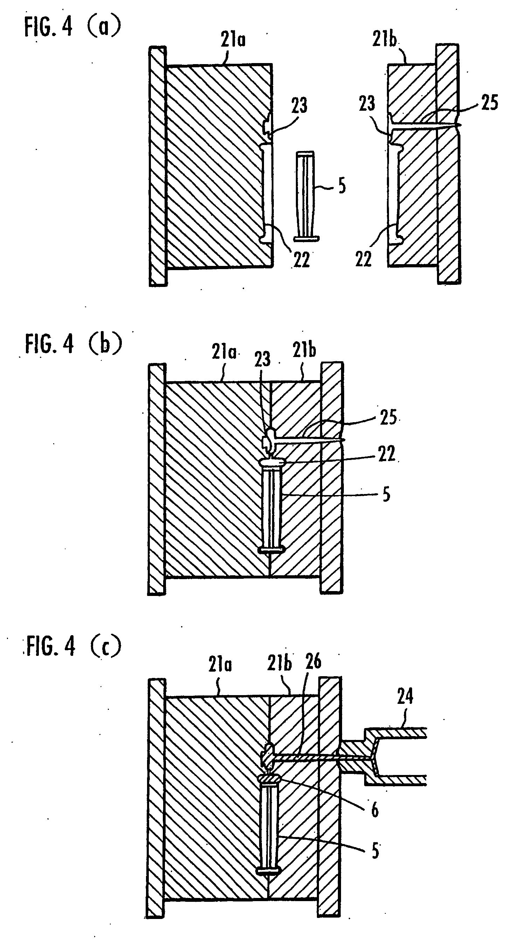

The plunger 5 and the gasket 6 may be attached by pressure in a heated state and joined together using the thermal fusion bonding process. Referring now to FIG. 4, one example of the thermal fusion bonding process will be described.

In the thermal fusion bonding process, a plunger 5 made of, for example, polypropylene is manufactured by a well-known injection molding method. Then, as shown in FIG. 4(a), the plunger 5 is placed in molds 21a and 21b. The molds 21a and 21b are provided with a cavity 22 which is formed in a shape matching the outer shape of the plunger 5 and the gasket 6, and a pinpoint gate 23 located in the middle on the surface of the gasket 6 which is to be formed. The mold 21b are provided with a runner portion 25 which connect the pinpoint gate 23 and a injection molding machine 24. The cavity 22 of the molds 21a and 21b is formed by performing an electric discharge digging machining, and the inner face is polished by using abrasive coating having an average particle size from 10 to 50 µm.

Subsequently, as shown in FIG. 4(b), the molds 21a and 21b having the plunger 5 placed are closed. Then, as shown in FIG. 4(c), the runner portion 25 of the mold 21b is connected to the injection molding machine 24, and, for example, S-EP-S based thermoplastic elastomer 26 is injected to the cavity 22 in a molten state. As the result, the plunger 5 and the gasket 6 are thermally fused by heat and pressure generated from the injection in a state of having the hole 12 of the gasket 6 fit into the base 10 of the plunger 5 so that the plunger 5 and the gasket 6 can be formed integrally.

The injection molding of the S-EP-S based thermoplastic elastomer 26 is preferably performed at a cylinder temperature ranging from 170 to 260°C and at a injection pressure ranging from 250 to 1500 kg/cm² in order to thermally fuse the plunger 5 and the gasket 6. In the injection molding, sometimes sufficient adhesion strength cannot be obtained when the cylinder temperature is lower than 170°C or an injection pressure is lower than 250 kg/ cm². On the contrary, when a cylinder temperature is higher than 260°C or an injection pressure is higher than 1500 kg/ cm², members which were placed in advance may be damaged by the resin which is injected afterward.

According to the present embodiment, an injection molding of S-EP-S based thermoplastic elastomer 26 was performed by using a general purpose machine of a screw plunger type as the injection molding machine 24, at the cylinder temperature of 210°C, at an injection pressure at peak from 250 to 1000 kg/ cm², and dwell pressure application at 20% of the injection pressure. As the result, the gasket 6 made of S-EP-S based thermoplastic elastomer 26 was thermally fused to the plunger 5 made of polypropylene, thereby integrally forming the plunger 5 and the gasket 6.

In the present embodiment, a pre-formed plunger 5 was placed in the molds 21a and 21b. However, the gasket 6 may be formed beforehand and placed in the molds 21a and 21b.

In this case, the gasket 6 which is placed in advance in the molds 21a and 22b is softer than the material for the plunger 5 which is filled later. Thus, the material of the plunger 5 is injected, for example, on condition that the cylinder temperature is from 190 to 230C° and the cylinder injection pressure is from 250 to 600 kg/ cm².

Instead of the device shown in FIGs. 4(a) to 4(c), a two-color mold such as an inverse type, core rotation type, core back type, or die slide type may be used to sequentially inject the material of the plunger 5 and the material of the plunger 6.

### INDUSTRIAL APPLICABILITY

The present invention may be utilized for a disposable injector for use as a medical device or the like.

## Claims

1. A injector comprising a cylindrical barrel to which a needle is to be attached, and a plunger having a gasket on the tip, which slides within the cylindrical barrel while maintaining an excellent adhesion to the inner wall surface of the injector, wherein
the gasket is a substantial discoid form of which the whole side face is a curved surface having a convex arc towards the inner wall surface along the axial direction of the plunger, and tightly contact the inner wall surface of the cylindrical barrel at a portion of the curved surface,
the gasket is joined to a base provided on the tip of the plunger via a hole provided on an end face of the gasket facing the plunger while leaving a flexible space on the plunger side, the base having a smaller diameter than the end face of the plunger,
an annular groove portion having a larger diameter than the base is provided on the surface of the plunger side, and a bending portion is provided on the outer peripheral side of the annular groove portion in which the bottom of the annular groove portion becomes a supporting point when the gasket slides along the axial direction of the cylindrical barrel, and the bend portion is formed so that the distance between the bottom of the annular groove portion and the peak of the curved side is smaller than the curvature radius of the curved side.

2. The injector according to claim 1, wherein said gasket consists of a synthetic resin having Shore (A) hardness from 20 to 70.

3. The injector according to claim 2, wherein said synthetic resin is one synthetic resin selected from the group consisting of polystyrene-based thermoplastic elastomer, polyolefin-based thermoplastic elastomer, polyester-based thermoplastic elastomer, polyamide-based thermoplastic elastomer, polyvinylchloride-based thermoplastic elastomer, and polystyrene-added styrene-butadiene rubber.
